# EUROPEAN PATENT APPLICATION

(11) **EP 3 192 546 A1**
(43) Date of publication of application: **19.07.2017**
(21) Application number: 16150824.7
(22) Date of filing: 12.01.2016
(51) Int. Cl.: A61M 5/315, A61M 5/20, A61M 5/24, A61M 5/31

(54) **DRUG DELIVERY DEVICE WITH IMPROVED DOSE ACCURACY**

(71) Applicant: NOVO NORDISK A/S, 2880 Bagsværd (DK)
(72) Inventor: The designation of the inventor has not yet been filed

(57) **Abstract**

Method of manufacturing a drug delivery device, comprising the steps of (i) providing a plurality of components which in an assembled state form a processor-controlled drug delivery device, (ii) determining a parameter value for at least one component, (iii) assembling the components to form a drug delivery device, and (iv) storing in the processor memory correction data, the correction data being based on one or more differences between a determined parameter value and a corresponding nominal value.

## Description

The present invention generally relates to drug delivery devices. In a specific aspect the invention relates to devices and methods providing improved dose accuracy.

### BACKGROUND OF THE INVENTION

In the disclosure of the present invention reference is mostly made to the treatment of diabetes by subcutaneous drug delivery, however, this is only an exemplary use of the present invention.

As medical science progress, more and more conditions can be treated and help people live longer. Some conditions last through-out the patient's lifetime, during which the patient is depending on some sort of medication. Such medication often has to be given as subcutaneous injections once or more often per day.

To perform such subcutaneous injections drug delivery devices in the form of drug injection devices have greatly improved the lives of patients who must self-administer drugs and biological agents. Drug injection devices may take many forms, including simple disposable devices that are little more than an ampoule with an injection means or they may be highly sophisticated electronically controlled instruments with numerous functions. Regardless of their form, they have proven to be great aids in assisting patients to self-administer injectable drugs and biological agents. They also greatly assist care givers in administering injectable medicines to those incapable of performing self-injections.

In particular pen-style injection devices have proven to provide an accurate, convenient, and often discrete, way to administer drugs and biological agents, such as insulin. While pen-style injection devices are typically cylindrically shaped with a mounted needle protruding from the most distal portion of one end of the device, some devices have other shapes with the needle no longer protruding from the most distal part of an end of the device.

Typically, injection devices use a pre-filled cartridge containing the medication of interest, e.g. 1.5 or 3.0 ml of insulin, GLP-1 or growth hormone. The cartridge is typically in the form of a generally cylindrical transparent glass cylinder having a distal bottle neck portion with a distal opening closed by a needle pierceable septum and an opposed proximal opening in which an elastomeric piston is received, the piston being arranged to be moved by the dosing mechanism of the injection device. The injection devices generally are of two types: "Durable" devices and "disposable" devices. A durable device is designed to allow a user to replace one cartridge with another cartridge, typically a new cartridge in place of an empty cartridge. In contrast, a disposable device is provided with an integrated cartridge which cannot be replaced by the user; when the cartridge is empty the entire device is discarded.

In order to improve convenience, user-friendliness and provide additional features, e.g. detection and storing of expelling data, drug delivery devices have been provided with electrically driven means, typically in the form of an electronically controlled motor driving a piston rod through a gear arrangement, e.g. as shown in US 6,514,230 and US 2011/306927.

Because more and more conditions can be treated by new drugs and some conditions seem to become both more frequent and more severe, the need for more injection devices and in some cases higher capacity devices grow. Diabetes is an example of such a condition. A rapidly increasing part of the world population gets diabetes and need treatment with insulin. In some parts of the world not only does a larger part of the population need insulin treatment, but a larger part needs larger and larger doses and in extreme cases, some patients needs the full contents of a disposable device each day.

The obvious solution to this situation would be to produce more devices or increase the drug content of the device. However, increasing the size of the drug content of the devices means increasing the size of the devices, which again leads to an increase of use of resources, production costs and becomes unhandy and less convenient for the user. Increasing the number of devices produced also means additional use of resources. Thus both of these suggested solutions would lead to an increase in the overall treatment costs, which seems like a natural consequence of an increase in treatment need, but an increase in treatment costs will also mean that a smaller part of the world population can afford being treated.

Naturally, by means of competition between manufacturers, the production costs of both drugs and devices are continually being lowered and to some extend countering the consequences of increasing demand for treatment.

However, the production costs of a device is often far larger than the production costs of the drug itself, and hence the treatment costs could also be lowered by increasing the concentration of the drug and thereby increase the capacity per device without increasing the size of the device. By increasing the concentration more patients can be treated requiring fewer produced devices, thereby lowering the cost ratio between drug and device, resulting in the ability to treat a larger part of the world population.

Since all types of medications introduce a principal risk of under- or overdosing, such devices must be able to accurately inject the intended dose. During injection the intended dose must be physically expelled from a reservoir and into the skin of the patient. The accuracy with which this operation can be carried out depends on the accuracy of the mechanical parts of the device. Since deviations between intended and actually administered dose presents a health risk, such devices must be produced within very strict tolerances.

The production costs of a device depends on the production costs of the parts in the device and the production costs of parts increase rapidly when acceptable tolerances are lowered. Therefore, an increase in concentration of the drug in a device will lead to a significant increase in production costs of the device, due to an increase in required production tolerances for a number of parts in the device, and may therefore not reduce the treatment costs as intended.

Addressing the issue of accuracy, electronically controlled motorized drug delivery devices have been proposed in which the individual device after assembly is calibrated to compensate for variations in the manufacture and assembly of various components of the device. After calibration a corresponding correction value is stored in the device and used to compensate for the measured deviation from the nominal specifications, see e.g. WO 2010/ 027636 and WO 2002/028456.

Having regard to the above, it is an object of the present invention to provide drug delivery devices and methods therefor which provide improved dose accuracy in a cost-effective way.

### DISCLOSURE OF THE INVENTION

In the disclosure of the present invention, embodiments and aspects will be described which will address one or more of the above objects or which will address objects apparent from the below disclosure as well as from the description of exemplary embodiments.

The above problem is addressed by compensating for the mechanical variations within the allowable tolerances by breaking the link between dose setting and actual dose expelling, such that the relationship between a set and an expelled dose is different between devices in order to compensate for differences in actual dimensions for components in the devices due to fabrication tolerances.

In presently available drug delivery devices, both manual and motorized, the relationship between a set dose and an expelled dose is based on nominal values of key components. Thus, the devices are designed to perform mechanically identical, while limiting variation in actually expelled volume by controlling tolerances/variations in component dimensions. Basically, the tolerances are used to limit variations in expelled volume for a given motion of the drug expelling mechanism.

Thus, in accordance with a general common aspect of the invention, a drug delivery device can be designed to work in the opposite way and use variations in actual component dimensions to adjust the way the device operate.

Correspondingly, the displayed dose amount during dose setting may be controlled so as to match the actual dose amount that will be expelled from a given specific device, or the expelling mechanism may be controlled so as to actually expel the nominally set dose amount.

The first concept may be implemented by providing a drug delivery device with an electronically controlled read-out of dose size during dose setting. For example, a correction table may be incorporated in the electronic circuitry and used to display the actual dose that will be injected at a specific dose setting instead of displaying a nominal dose size corresponding to the dose setting in question. Thereby, the user may automatically and without being aware, compensate for the inaccuracy caused by the tolerances in the device, by setting the dose size based on the read-out of actual expected dose size from the display, instead of setting the dose size based on a nominal intended dose size release by a fixed ratio between dose size dial and (mechanical) read-out of nominally expected dose size.

The second concept may be implemented by providing a drug delivery device with an electronically controlled expelling mechanism and by implementing a correction table in the control circuitry of each device individually. Thereby the expelling mechanism can be adjusted to compensate for the variations in component dimensions of a given specific device and thereby make the actual expelled dose match the intended nominal dose setting.

Both concepts require a correction table to be established for each individual device. To provide each individual device with such a correction table, one or more dimensions or parameters for one or more key components for a given device are determined individually and key parameters are captured as well as the components location in the assembly line during assembly of the device. The values for a given member may be determined by e.g. measurement or by identifying an identifier, e.g. mould code, for which the values are known.

When a device is assembled, it is then known which specific components the device is made from and their key parameters are known. Thus the variation between a nominal set dose and an actual injected dose can be calculated and a correction table can be loaded into the electronics of the device and handled accordingly, whether the device is designed based on the first or second concept. Indeed, theoretically, the two concepts could be combined.

Thus the invention allows the production of a device with more accurate and uniform performance, without requiring any new or more accurately produced components, which would increase costs significantly. Furthermore, the electronic compensation of mechanical inaccuracies also allows for the compensation of non-linear behaviour of inaccuracies or variations of cartridge diameter along the cartridge length, which would be impossible to compensate for mechanically.

If implemented corresponding to the above-described second concept, an electronic read-out and thus the electronic display can be omitted, but the implementation of an electronically controlled expelling mechanism would be necessary. However, the costs of implementing such a drive unit may well be comparable with the costs of reducing the allowable tolerances of a single component, which would be necessary but not sufficient to reduce variations between intended and actual expelled dose significantly.

Both of the above-described concepts allow the concentration of the drug in the device to be increased and thus fewer devices (if prefilled) or cartridges are needed to meet an increasing demand, thereby lowering the required number of device or cartridges necessary to provide treatment for a given population. Since the costs of production of a device is significant compared to the costs of production of the drug itself, an increase in concentration will also lead to a reduction in production costs per unit of drug in a device.

Thus, in accordance with a **first aspect** of the invention a **method of manufacturing a drug delivery device** is provided, comprising the steps of (i) providing a plurality of components which in an assembled state form a processor-controlled drug delivery device comprising dose setting means, drug expelling means adapted to expel a user-set dose amount of drug, and a processor with memory, the drug delivery device comprising a drug-filled cartridge or means for receiving a drug-filled cartridge, the cartridge comprising an outlet and an axially displaceable piston, the components comprising a first group of components adapted to move during drug expelling, the first group of components comprising an axially displaceable piston drive member adapted to engage the piston of a cartridge, and a second group of components adapted to be stationary during drug expelling but interfacing with a member of the first group, (ii) determining a parameter value for at least one of: a member of the first group, and a member of the second group, (iii) assembling the components to form a drug delivery device, and (iv) storing in the processor memory correction data, the correction data being based on one or more differences between a determined parameter value and a corresponding nominal value. A component may be in the form of an assembly comprising a number of individual components. A parameter value may be "dimensional", e.g. a measured length or diameter, or "functional", e.g. a measured axial output for a given rotational input.

In this way a drug delivery device is provided cost-effectively with means allowing the processor, based on the correction value and a nominally set dose amount, to (i) calculate a corrected axial displacement for the piston drive member and control the expelling means to move the piston drive member corresponding to the corrected axial displacement, or (ii) calculate a corrected dose amount and control a display to display the corrected dose amount, whereby an expelled and a displayed dose amount correspond to each other.

To address the issue that a given parameter may vary for a given member at least two parameter values may be determined for a given member as a function of a given dimension, e.g. along the length of the member such as a piston rod or a cartridge, or the position, e.g. a rotational position for an expelling assembly. Correspondingly, the correction data may comprise a number of correction values varying as a function of a state of the drug delivery device. The state may for example be the actual position of the piston rod or the dose size actually set.

The plurality of components in the assembled state may form a drug delivery device as defined below.

In accordance with a **further aspect** of the invention a **method of manufacturing a drug delivery assembly** is provided, comprising the steps of (i) providing a drug-filled cartridge, the cartridge comprising an outlet and an axially displaceable piston, (ii) determining at least one parameter value for the cartridge, (iii) providing the cartridge with readable information in respect of the determined parameter value(s), and (iv) providing a processor-controlled drug delivery device comprising a cartridge holder adapted to receive the cartridge, means for reading the cartridge readable information, an expelling assembly, and a processor with memory.

The method may comprise the further steps of inserting the cartridge in the cartridge holder, reading the cartridge readable information, and storing in the processor memory correction data, the correction data being based on a difference between the determined parameter value and a corresponding nominal value. The "manufacturing" step of inserting the cartridge in the cartridge holder may be performed by the user during normal use of the device, e.g. when replacing a cartridge. The drug delivery device may be provided with one or more features from the below-described exemplary embodiments of a drug delivery devices.

In accordance with a yet **further aspect** of the invention a **method of manufacturing a drug cartridge** is provided, comprising the steps of (i) providing a drug-filled cartridge, the cartridge comprising an outlet and an axially displaceable piston, (ii) determining at least one parameter value for the cartridge, and (iii) providing the cartridge with readable information in respect of the determined parameter value. The provided cartridge may be used in one of the above-described methods or in one of the below-described exemplary embodiments of a drug delivery device.

In general, the above-described steps of determining a parameter value for a given member or component may be in respect of a given dimension for which a corresponding nominal value has been specified.

In accordance with a **general aspect** of the invention a **drug delivery device** is provided comprising a drug-filled cartridge or means for receiving a drug-filled cartridge, the cartridge comprising an outlet and an axially displaceable piston. The drug delivery device is provided with an expelling assembly comprising an axially displaceable piston drive member adapted to engage the piston of a cartridge, dose setting means allowing a user to set a nominal dose amount of drug to be expelled, a drive mechanism adapted to move the piston drive member a nominal distance corresponding to a set nominal dose amount, and a display adapted to display a dose amount value. The drug delivery device further comprises a processor with a memory in which a correction value is stored, the processor being adapted to, based on the correction value and a nominally set dose amount, (i) calculate a corrected axial displacement for the piston drive member and control the drive mechanism to move the piston drive member corresponding to the corrected axial displacement, or (ii) calculate a corrected dose amount and control the display to display the corrected dose amount, whereby the expelled and display dose amount correspond to each other.

At least two correction values may be stored in the memory, the correction values being correlated to the position of the piston rod, the processor being adapted to, based on the correction value for the current position of the piston rod and a nominally set dose amount, calculate a corrected dose amount and control the display to display the corrected dose amount.

In accordance with a **specific aspect** of the invention a drug delivery device is provided comprising a drug-filled cartridge or means for receiving a drug-filled cartridge, the cartridge comprising an outlet and an axially displaceable piston, and an expelling assembly. The expelling assembly comprises a first group of components adapted to move during drug expelling, the first group of components comprising an axially displaceable piston drive member adapted to engage the piston of a cartridge, and a second group of components adapted to be stationary during drug expelling but interfacing with a member of the first group. The expelling assembly further comprises dose setting means allowing a user to set a nominal dose amount of drug to be expelled, a display adapted to display a dose amount, drive means for moving the piston drive member in a distal direction to thereby expel drug from an inserted cartridge, and a processor comprising a memory, the processor being adapted to **control the display** to display a dose amount. A correction value is stored in the memory, the correction value being based on one or more differences between a determined parameter value and a corresponding nominal value for at least one of: a member of the first group, a member of the second group, and a received cartridge. The processor is adapted to, based on the correction value and a nominally set dose amount, calculate a corrected dose amount and control the display to display the corrected dose amount.

The display may be adapted to display dose amounts in increments of a given unit, e.g. rounded to a desired precision such as 1 unit of insulin (IU). The dose setting means may be adapted to set a nominal dose amount of drug to be expelled in increments which are a fraction of the display increment, this allowing a discrepancy between a set dose and a displayed dose to be "masked". For example, if the display displays dose amounts in full IU the dose setting means may have increments of 0.25 IU such that it is less apparent that there not necessarily is a 1:1 relation between what is nominally set and what is actually displayed - and subsequently expelled.

The drug delivery device may comprise a cartridge holder adapted to receive a cartridge, the cartridge being provided with readable information in respect of a determined parameter value for the cartridge, and means for reading the cartridge readable information, wherein the processor is adapted to calculate a correction value based at least in part on the difference between the cartridge parameter value information and a corresponding stored nominal value.

In accordance with a **further specific aspect** of the invention a drug delivery device is provided comprising a drug-filled cartridge or means for receiving a drug-filled cartridge, the cartridge comprising an outlet and an axially displaceable piston, and an expelling assembly. The expelling assembly comprises a first group of components adapted to move during drug expelling, the first group of components comprising an axially displaceable piston drive member adapted to engage the piston of a cartridge, and a second group of components adapted to be stationary during drug expelling but interfacing with a member of the first group. The expelling assembly further comprises dose setting means allowing a user to set a nominal dose amount of drug to be expelled, the nominal dose amount corresponding to a nominal axial displacement for the piston drive member, a display adapted to display a dose amount, a motor for moving the piston drive member in a distal direction to thereby expel drug from an inserted cartridge, and a processor comprising a memory, the processor being adapted to control the motor to displace the piston drive member axially. A correction value is stored in the memory, the correction value being based on one or more differences between a determined parameter value and a corresponding nominal value for at least one of: a member of the first group, a member of the second group, and a received cartridge. The processor is adapted to, based on the correction value and a nominally set dose amount, calculate a corrected axial displacement for the piston drive member and control the motor to move the drive member corresponding to the corrected axial displacement. In this way the actually expelled dose amount of drug corresponds more precisely to the nominally set dose amount.

At least two correction values may be stored in the memory, the correction values being correlated to the position of the piston rod, the processor being adapted to, based on the correction value for the current position of the piston rod and a nominally set dose amount, calculate a corrected axial displacement for the piston drive member and control the motor to move the drive member corresponding to the corrected axial displacement.

As used herein, the term "drug" is meant to encompass any flowable medicine formulation capable of being passed through a delivery means such as a cannula or hollow needle in a controlled manner, such as a liquid, solution, gel or fine suspension, and containing one or more drug agents. Representative drugs include pharmaceuticals such as peptides (e.g. insulins, insulin containing drugs, GLP-1 containing drugs as well as derivatives thereof), proteins, and hormones, biologically derived or active agents, hormonal and gene based agents, nutritional formulas and other substances in both solid (dispensed) or liquid form. In the description of the exemplary embodiments reference will be made to the use of insulin containing drugs, this including analogues thereof as well as combinations with one or more other drugs.

### BRIEF DESCRIPTION OF THE DRAWINGS

In the following exemplary embodiments of the invention will be described with reference to the drawings, wherein
fig. 1A shows a pen-formed drug delivery device,
fig. 1B shows the pen device of fig. 1A with the pen cap removed,
fig. 2 shows a drug delivery device of the same general type as in fig. 1A but provided with electronic circuitry and a display,
fig. 3 shows an expelling assembly,
fig. 4 shows a piston rod,
fig. 5 shows a drug cartridge,
figs. 6A-6E show maximum deviations between dialled and given dose for different configurations of a drug delivery device,
figs. 7A and 7B show maximum deviations between dialled and given dose for different configurations of drug delivery devices provided with electronic display compensation, and
figs. 7C and 7D show maximum deviations between dialled and given dose for different configurations of drug delivery devices provided with electronic motor drive compensation.

In the figures like structures are mainly identified by like reference numerals.

### DESCRIPTION OF EXEMPLARY EMBODIMENTS

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member or element is used for a given component it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessary can be assembled to provide a unitary or functional assembly during a given assembly procedure but is merely used to describe components grouped together as being functionally more closely related.

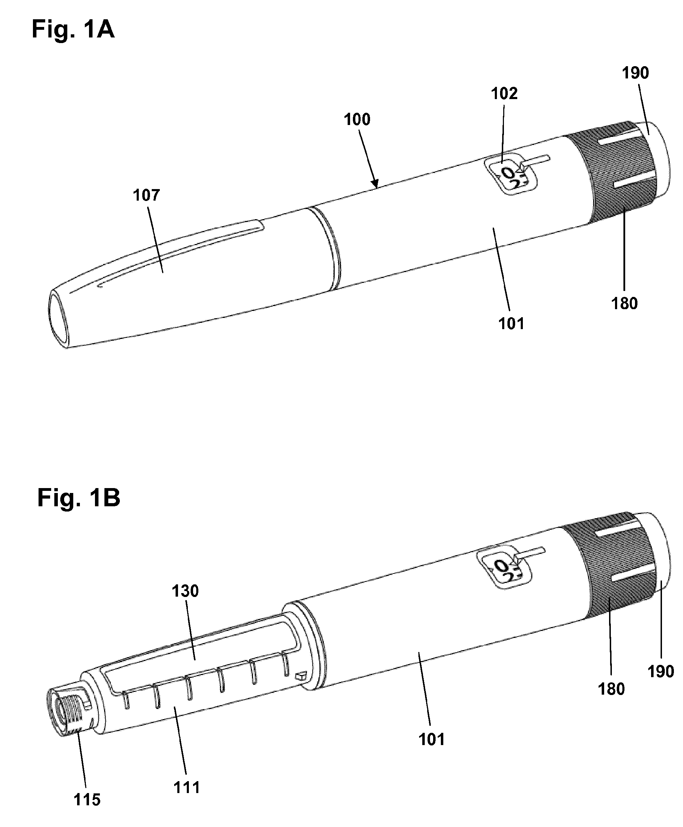

Before turning to embodiments of the present invention *per* se, an example of a pre-filled drug delivery will be described, such a device providing the basis for an exemplary embodiment of the present invention. Although the pen-formed drug delivery device 100 shown in figs. 1A and 1B may represent a "generic" drug delivery device, the actually shown device is a FlexTouch® pre-filled drug delivery pen as manufactured and sold by Novo Nordisk A/S, Bagsværd, Denmark.

The pen device 100 comprises a cap part 107 and a main part having a proximal body or drive assembly portion with a housing 101 in which a drug expelling mechanism is arranged or integrated, and a distal cartridge holder portion 111 in which a drug-filled transparent cartridge 130 with a distal needle-penetrable septum is arranged and retained in place by a non-removable cartridge holder attached to the proximal portion, the cartridge holder having openings allowing a portion of the cartridge to be inspected as well as distal coupling means 115 allowing a needle assembly to be releasably mounted. The cartridge is provided with a piston driven by a piston rod forming part of the expelling mechanism and may for example contain an insulin, GLP-1 or growth hormone formulation. The mechanism comprises a scale drum member provided with a plurality of dose size indices, the scale drum member being arranged rotationally corresponding to the general axis. The housing comprises a display opening (or window) 102 arranged to show a scale drum member dose size indicia corresponding to a set dose. A proximal-most rotatable dose setting member 180 serves to manually set a desired dose of drug shown in display window 102 and which can then be expelled when the button 190 is actuated. Depending on the type of expelling mechanism embodied in the drug delivery device, the expelling mechanism may comprise a spring as in the shown embodiment which is strained during dose setting and then released to drive the piston rod when the release button is actuated. Alternatively the expelling mechanism may be fully manual in which case the dose member and the actuation button moves proximally during dose setting corresponding to the set dose size, and then is moved distally by the user to expel the set dose, e.g. as in a FlexPen® manufactured and sold by Novo Nordisk A/S.

Although figs. 1A and 1B show a drug delivery device of the pre-filled type, i.e. it is supplied with a pre-mounted cartridge and is to be discarded when the cartridge has been emptied, in alternative embodiments the drug delivery device may be designed to allow a loaded cartridge to be replaced, e.g. in the form of a "rear-loaded" drug delivery device in which the cartridge holder is adapted to be removed from the device main portion, or alternatively in the form of a "front-loaded" device in which a cartridge is inserted through a distal opening in the cartridge holder which is non-removable attached to the main part of the device.

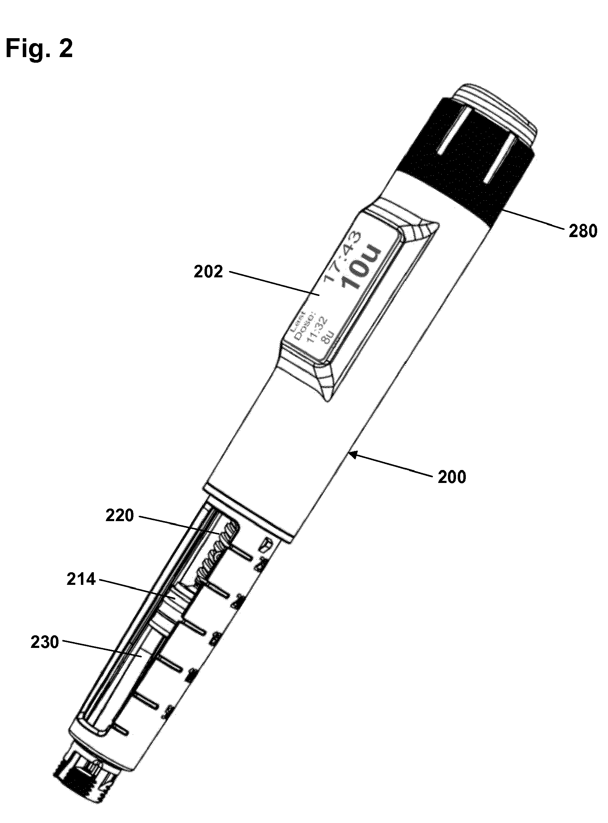

Fig. 2 shows a drug delivery device 200 having the same general appearance and being provided with essentially the same type of dose setting and expelling mechanism, however, the device is provided with electronic circuitry comprising a processor with memory, a display controlled by the processor and a sensor adapted to detect the size of a nominally set dose, i.e. the dose set by the user. In the figure a cartridge 230 provided with a piston 214 driven by a piston rod 220 can be seen.

If the drug delivery device 200 is a disposable prefilled device it is important that the electronic circuitry is provided using cost-effective technologies which is not necessarily designed for a long in-use time. For example, the display may be a printed display formed on a flexible carrier which may also form a substrate for further components being provided as "printed electronics", e.g. processor, memory, sensors, energy source. Alternatively one or more of these components may be in the form of discrete components mounted on the flexible carrier. An example of a pre-filled drug delivery device provided with a low-cost "electronic label" is described in WO 2015/071354. In the shown embodiment the scale drum is not used to show a set dose size, however, it is provided with a plurality of markings or codes which can be detected by the dose size sensors as the scale drum rotates helically during dose setting. The markings may be adapted to be detected by capacitive sensors which e.g. may be formed by a printing process on the flexible substrate. In case the drug delivery device 200 represents a durable device adapted to allow cartridge change, then the electronic circuitry may be provided with more traditional components such as an LCD.

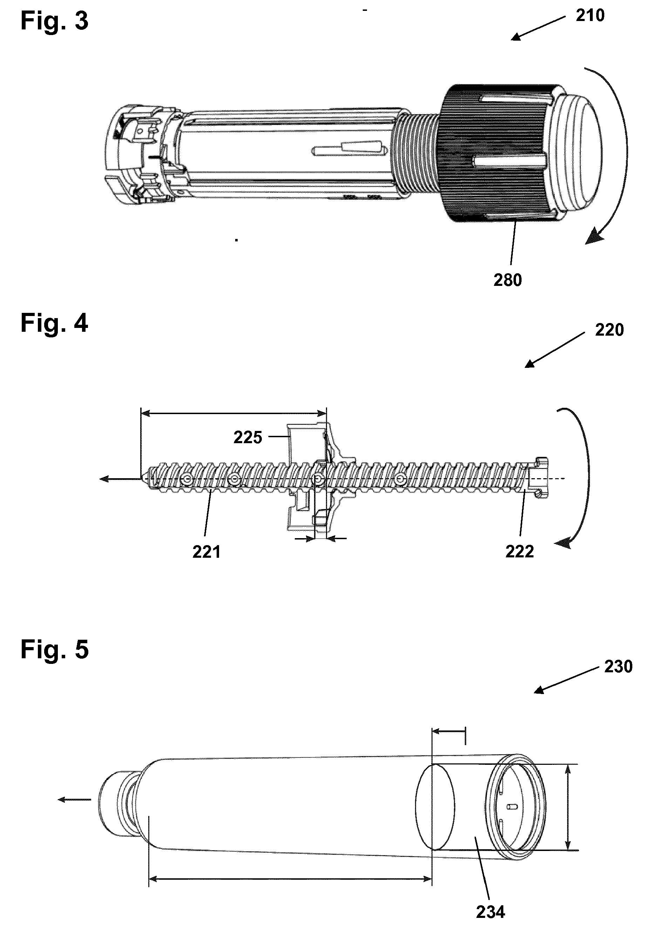

With reference to figs. 3-5 a number of components which can be used to realize the invention will be described, the figures showing three components of the drug delivery device 200 for which one or more parameter values can be determined during an assembly process.

More specifically, fig. 3 shows a dose setting and expelling unit 210 ("motor unit"), fig. 4 shows a piston rod 220 adapted to be rotationally driven by the motor unit, and fig. 5 shows a drug-filled cartridge 230 with an axially displaceable piston 234 adapted to be axially driven by the piston rod.

The motor unit 210 is set by rotating the rotatable dose setting member 280 which results in the drive "output" member being rotated correspondingly, however, due to tolerances variations will inevitably occur. The shown motor unit can be set in increments of 15 degrees for a total of 80 increments. Thus during manufacturing a given motor unit may be set in a number of rotational positions (i.e. up to 80) and the corresponding rotation of the output member may be determined and subsequently stored. As appears, to provide such data may be relatively costly. To reduce the amount of data one or more averages may be calculated for a number of dose size ranges.

For the piston rod 220 the pitch of each thread 221 along the length thereof may be measured. Alternatively, the pitch variation for a given piston rod having a specific location in a multi-cavity mould tool may be known from measurement of the mould, each cavity being provided with a code which can be identified, e.g. a code on the piston rod head 222. Indeed, by this method variations introduced by the mould process *per se* will not be detected. To reduce the amount of data one or more averages may be calculated for a number of sections of the piston rod. The shown housing nut portion 225 is not part of the piston rod.

For the cartridge 230 the diameter along the length thereof may be measured. For ease of measurement the external diameter may be determined. Indeed, by this method variations introduced by the thickness of the glass wall will not be detected. To reduce the amount of data one or more averages may be calculated for a number of sections of the cartridge. As disclosed above, if a given cartridge is to be used in a durable device provided with a corresponding reader, the cartridge may be provided with readable information in respect of the determined parameter value(s), i.e. in the form of a printed label.

After or during assembly of a given device correction data can be stored in the device processor memory, the correction data being based on one or more differences between a determined parameter value and a corresponding nominal value. For example, if the piston rod and the cartridge have been measured a correction table may be calculated, the table having correction values as a function of piston rod position. The actual position of the piston rod may be determined by e.g. detection of scale drum rotation as described above. For a given device the variations of the two components along the length thereof may for some positions cancel each other whereas for other positions they may be added. In addition, also the dose size will influence the calculated correction for a given expelled dose.

For a motor driven drug delivery device the basic concept is the same as for the above-described spring-driven device although the components having a "relevant" variability may not be the same. For example, the piston rod may be manufactured by machining and may thus have stricter tolerances. As a motor driven device will be designed to allow the cartridge to be replaced, a measured cartridge would have to be provided with a readable code and the device with a corresponding code reader. In contrast to the above-described spring-driven device it would be possible to use the correction data to control the motor such that the nominally set dose amount is very close to the dose actually expelled. Examples of motor-driven drug delivery devices which may be used as a platform for this aspect of the present invention are described in e.g. EP 2015/065544.

In the following aspects of the present invention will be illustrated by a number of examples.

### 1A. Reference drug delivery device example

In an exemplary drug delivery device the cartridge has an inner nominal diameter of 9,25 mm with a tolerance of +/- 0,1 mm corresponding to an inner cross section area of 67,2006 +/-1,4608 mm². The piston rod has nominal thread inclination of 3,6 +/- 0,04 mm/rev corresponding to a linear movement per degree of rotation of 0,01 +/- 0,00011111 mm/deg. The nominal offset is 0 +/- 0,02 mm. Drug concentration is 0,1 IU/mm³.

Standard and Regulations requirements are for dose sizes in the range 0-20 units (IU) set to +/- 1 IU, and for dose sizes larger than 20 IU +/- 5%.

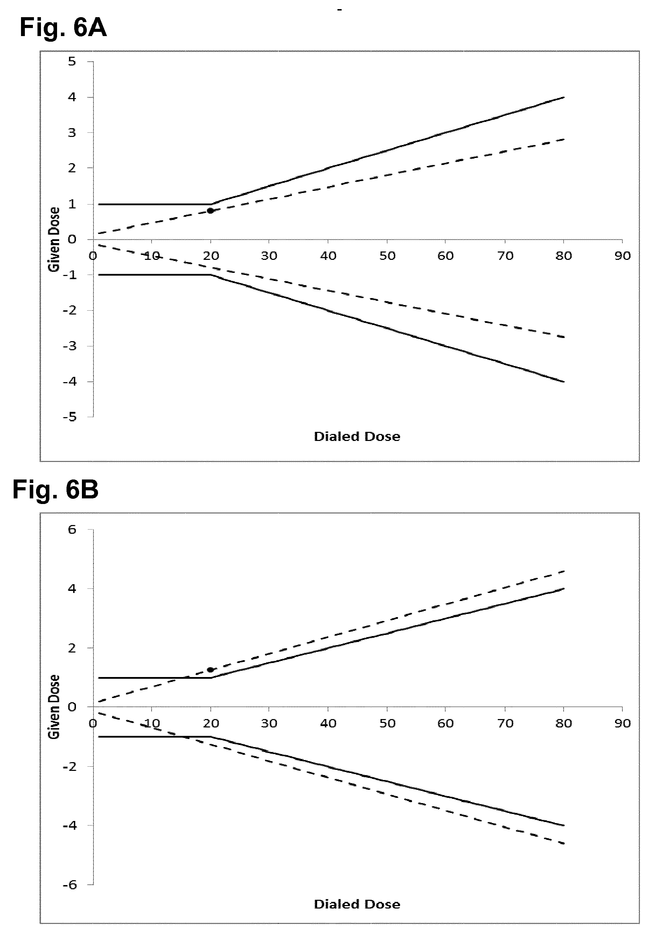

Based on the above dimensions and tolerances deviations between dialled and expelled doses can be calculated and are illustrated in fig. 6A in which the full line represents standard and regulation requirements and the dotted line represents maximum device variations due to mechanical tolerances. As seen in the figure an out-dosing of 20 IU can get closest to allowable limits. Thus, an out-dosing of 20 IU will be used in the following examples and illustrations.

For a drug delivery in which the piston rod rotates 15 degrees per increment of 1 IU dialling 20 IU will result in a piston rod rotation of 300 degrees.

| Linear movement of piston: | | | | | | | | |
|---|---|---|---|---|---|---|---|---|
| Nominal | 300 | degrees x | 0,01 | mm/deg | | | 3 | mm |
| Max. | 300 | degrees x | 0,01011111 | mm/deg | + Offset | | 3,053333 | mm |
| | | | | | | | | |

| Volume displacement of piston: | | | | | | | | |
|---|---|---|---|---|---|---|---|---|
| Nominal | 3 | mm x | 67,2006 | mm² | | | 201,6019 | mm³ |
| Max. | 3,053333 | mm x | 68,6615 | mm² | | | 209,6464 | mm³ |
| | | | | | | | | |

| Dose size of displaced volume : | | | | | | | | |
|---|---|---|---|---|---|---|---|---|
| Nominal | 201,6019 | mm3 x | 0,1 | IU/mm³ | | | 20,16 | IU |
| Max. | 209,6464 | mm3 x | 0,1 | IU/mm³ | | | 20,96 | IU |
| | | | | | | | | |

| Dose size deviation from nominal dose : | | | | | | | | |
|---|---|---|---|---|---|---|---|---|
| | | | 20,96 | - | 20,16 | = | 0,80 | IU |

As appears, dialling 20 IU may within the limits of the allowed tolerances result in an over-dosing of 0,8 IU which is thus acceptable within the +/- 1 IU tolerance limits for doses up to 20 IU.

### 1B: Example for drug delivery device with double cartridge inner diameter tolerances

An exemplary drug delivery device has a cartridge with an inner nominal diameter of 9,25 mm with a tolerance of +/- 0,2 mm corresponding to an inner cross section area of 67,2006 +/- 2,9374 mm². Otherwise the values are the same as in example A1.

Based on the above dimensions and tolerances deviations between dialled and expelled doses can be calculated and are illustrated in fig. 6B in which the full line represents standard and regulation requirements and the dotted line represents maximum device variations due to mechanical tolerances. As seen in the figure, doubling the cartridge diameter tolerance will bring performance variations well outside allowed limits.

Corresponding to the calculation in example A1 a maximum dose size deviation for a nominal 20 IU dose will be (21,42 - 20,16) IU = 1,26 IU.

### 1C: Example for drug delivery device with double piston rod inclination tolerances

An exemplary drug delivery device has a piston rod with nominal thread inclination of 3,6 +/-0,08 mm/rev corresponding to a linear movement per degree of rotation of 0,01 +/-0,00022222 mm/deg, and a nominal offset of 0 +/- 0,04 mm. Otherwise the values are the same as in example A1.

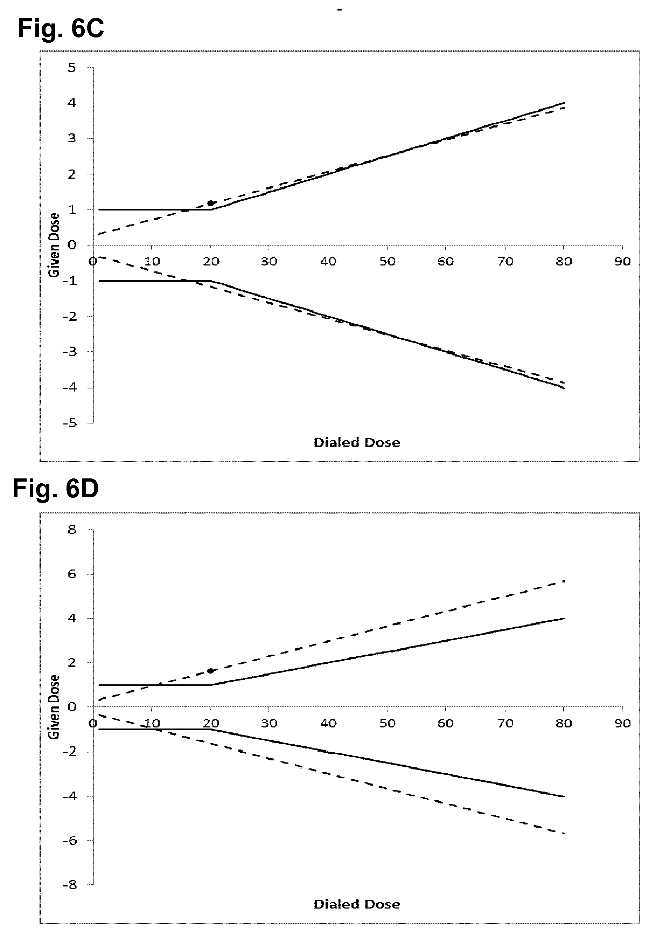

Based on the above dimensions and tolerances deviations between dialled and expelled doses can be calculated and are illustrated in fig. 6C in which the full line represents standard and regulation requirements and the dotted line represents maximum device variations due to mechanical tolerances. As seen in the figure, doubling the piston rod inclination tolerance will bring performance variations well outside allowed limits. However, not quite as much as if the cartridge inner diameter was doubled.

Corresponding to the calculation in example A1 a maximum dose size deviation for a nominal 20 IU dose will be (21,33 - 20,16) IU = 1,17 IU.

### 1D: Example for drug delivery device with double mechanical tolerances

An exemplary drug delivery device has a cartridge with an inner nominal diameter of 9,25 mm with a tolerance of +/- 0,2 mm corresponding to an inner cross section area of 67,2006 +/- 2,9374 mm², as well as a piston rod with nominal thread inclination of 3,6 +/- 0,08 mm/rev corresponding to a linear movement per degree of rotation of 0,01 +/- 0,00022222 mm/deg, and a nominal offset of 0 +/- 0,04 mm. Otherwise the values are the same as in example A1.

Based on the above dimensions and tolerances deviations between dialled and expelled doses can be calculated and are illustrated in fig. 6D in which the full line represents standard and regulation requirements and the dotted line represents maximum device variations due to mechanical tolerances. As seen in the figure, doubling the mechanical tolerances for both cartridge and piston rod will bring performance variations well outside allowed limits.

Corresponding to the calculation in example A1 a maximum dose size deviation for a nominal 20 IU dose will be (21,79 - 20,16) IU = 1,63 IU.

### 1E: Example for drug delivery device with triple drug concentration

In an exemplary drug delivery device the cartridge drug concentration is 0,3 IU/mm³. Correspondingly, the device has a piston rod with nominal thread inclination of 1,2 +/- 0,04 mm/rev corresponding to a linear movement per degree of rotation of 0,00333333 +/- 0,00011111 mm/deg, and a nominal offset of 0 +/- 0,02 mm. Otherwise the values are the same as in example A1.

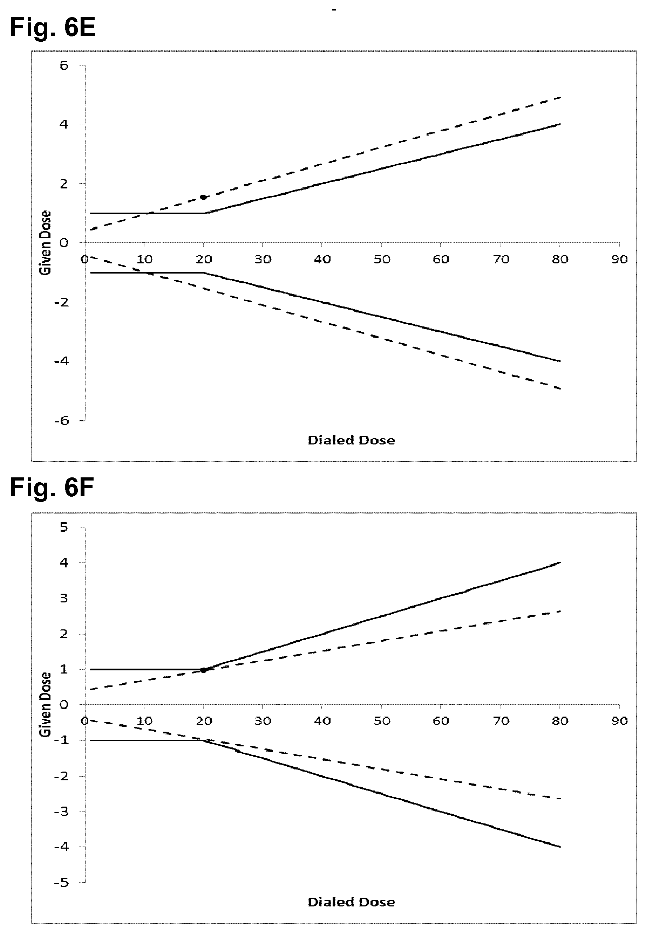

Based on the above drug concentration, dimensions and tolerances deviations between dialled and expelled doses can be calculated and are illustrated in fig. 6E in which the full line represents standard and regulation requirements and the dotted line represents maximum device variations due to mechanical tolerances. As seen in the figure, maintaining the mechanical tolerances and tripling the drug concentration will bring performance variations far outside allowed limits.

Corresponding to the calculation in example A1 a maximum dose size deviation for a nominal 20 IU dose will be (21,70 - 20,16) IU = 1,54 IU.

### 1F: Example for drug delivery device with triple drug concentration and compensation by reduced tolerances

In an exemplary drug delivery device the cartridge drug concentration is 0,3 IU/mm³. To compensate for the higher drug concentration the cartridge has an inner nominal diameter of 9,25 mm with a tolerance of +/- 0,05 mm corresponding to an inner cross section area of 67,2006 +/- 0,7285 mm². The piston rod has nominal thread inclination of 1,2 +/- 0,02 mm/rev corresponding to a linear movement per degree of rotation of 0,00333333 +/-0,00005556 mm/deg. The nominal offset is 0 +/- 0,02 mm.

Based on the above drug concentration, dimensions and tolerances deviations between dialled and expelled doses can be calculated and are illustrated in fig. 6F in which the full line represents standard and regulation requirements and the dotted line represents maximum device variations due to mechanical tolerances. As seen in the figure, tripling the drug concentration requires the mechanical tolerances reduced to less than half to keep performance variations just inside allowed limits.

Corresponding to the calculation in example A1 a maximum dose size deviation for a nominal 20 IU dose will be (21,13 - 20,16) IU = 0,97 IU.

As illustrated by the above examples decreasing tolerances and/or increasing drug concentration will bring the possible performance variations outside allowed limits. Correspondingly, in the following examples will be given which will illustrate how aspects of the present invention can be used to reduce performance variations for a given drug delivery device having given tolerances.

### 2A: Example for drug delivery device with double mechanical tolerances and electronic display compensation

An exemplary drug delivery device has a cartridge, piston rod and drug concentration with dimensions and tolerances as set out in example 1D above, each increment of 1 IU corresponding to a piston rod rotation of 15 degrees.

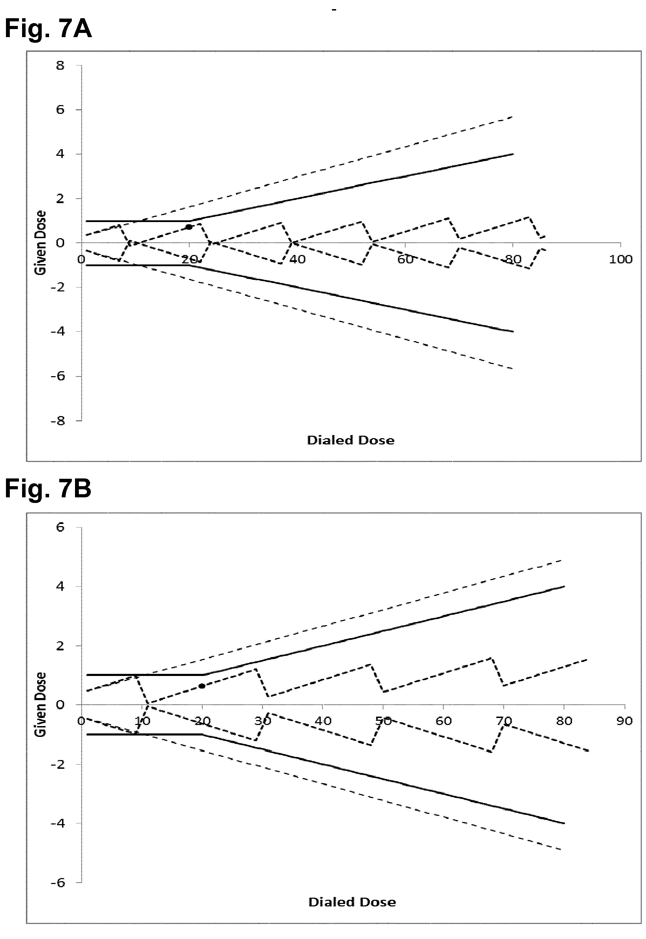

Based on the above drug concentration, dimensions and tolerances deviations between dialled and expelled doses can be calculated and are illustrated in fig. 7A in which the full line represents standard and regulation requirements, the dotted line represents maximum device variations due to mechanical tolerances.

However, in accordance with an aspect of the present invention the drug delivery device is provided with an electronically controlled display that will compensate for the difference between the nominally set value and the calculated value for the drug amount to be expelled. More specifically, the calculated value for a given set dose size will be rounded down to the nearest integer value which will then be displayed, e.g. a dose of 47 IU is dialled, a dose of 50,5 IU is calculated, and a dose 50 IU is displayed, this resulting in an over-dosing deviation of 0,5 IU. In fig. 7A the bold dotted line shows variations in out-dosing compared to displayed values. As also seen in fig. 7A, the electronic display compensation reduces the actual variations in out-dosing to less than the reference example, even if the mechanical tolerances have been doubled.

Fig. 7A illustrates maximum variations, however, a given component such as a cartridge or piston rod will not have maximum tolerances over the entire dose range.

As dimensions for both cartridge inner diameter and piston rod inclination may vary along the length of the members, it would be necessary for optimal compensation to determine the actual dimensions along the length of the members with a high resolution. However, for practical purposes the dimensions may be determined as an average for a number of ranges, e.g. 10 ranges for the entire drug volume of e.g. 300 IU of insulin.

In the following example dimensions for an exemplary device has been determined which are representative for a dose of 20 IU being expelled from a given device in a given state, i.e. the 20 IU may correspond to the piston rod in the initial position or any other position in which 20 IU can be expelled from the cartridge. The dimensions are imaginary and thus not based on actual measurements.

### Measurement values

Inner diameter: 9,44 mm
Inclination of threading: 3,67 mm/rev.

### Calculation results

Cross section area: 69,98965777 mm²
Actual piston movement/click: 0,152916667 mm
Actual out-dosing/click: 1,070258517 IU
Display Correction factor: 1,070258517

Correspondingly, when the user sets a dose of 19 IU this will correspond to 19 IU x 1,070258517 = 20,33 IU being expelled, which will be rounded and displayed as 20 IU, a deviation of 0,33 IU.

### 2B: Example for drug delivery device with triple drug concentration and electronic display compensation

An exemplary drug delivery device has a cartridge, piston rod and drug concentration with dimensions and tolerances as set out in example 1E above, each increment of 1 IU corresponding to a piston rod rotation of 15 degrees. Corresponding to example 2A the drug delivery device is provided with an electronically controlled display that will compensate for the difference between the nominally set value and the calculated value for the drug amount to be expelled by rounding down the calculated value for a given set dose size to the nearest integer value which will then be displayed.

Based on the above drug concentration, dimensions and tolerances deviations between dialled and expelled doses can be calculated and are illustrated in fig. 7B in which the full line represents standard and regulation requirements, the dotted line represents maximum device variations due to mechanical tolerances, and the bold dotted line shows variations in out-dosing compared to displayed values. As also seen, an increase in concentration may require clicks for half units to reduce rounding errors.

### 2C: Example for drug delivery device with double mechanical tolerances and electronic piston rod drive

An exemplary drug delivery device has a cartridge, piston rod and drug concentration with dimensions and tolerances as set out in example 1D above, each increment of 1 IU corresponding to a piston rod rotation of 15 degrees.

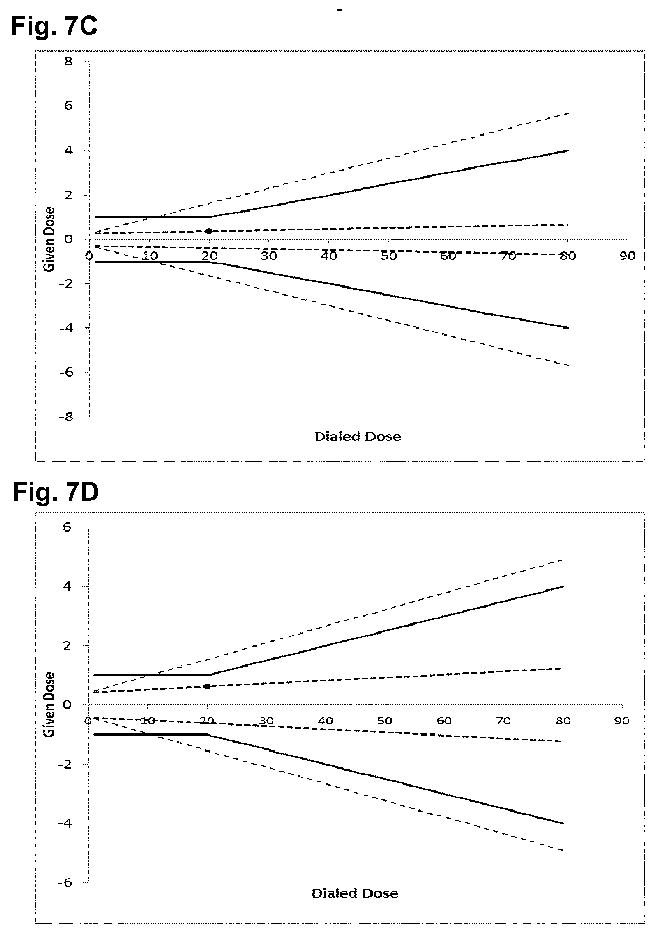

Based on the above drug concentration, dimensions and tolerances deviations between dialled and expelled doses can be calculated and are illustrated in fig. 7C in which the full line represents standard and regulation requirements, the dotted line represents maximum device variations due to mechanical tolerances.

However, in accordance with a further aspect of the present invention the drug delivery device is provided with an electronically controlled motor drive that will adjust the amount of piston rod rotation for a given nominally set dose size such that the expelled amount of drug corresponds to the set amount of drug. In example 2A above a display correction factor of 1,070258517 was calculated. The corresponding piston rod turn correction factor for the same measured values is the inverse value thereof: 0,934354.

### Calculation example for a set dose of 20 IU

Nominal turn angle for piston rod: 20 x 15 deg = 300 deg
Actual piston rod rotation: (300 deg x 0,934354)+1 = 281,306 mm/deg
(A worst case inaccuracy of the motor drive of +/- 1,0 deg is assumed)
Actual piston movement: (281,3061 deg x 0,01019 mm/deg)+0,04 mm = 2,908 mm
(A worst case offset due to elasticity and friction of 0,04 mm is assumed)
Actual volume displaced: 2,908 mm x (π x (9,45mm/2)² = 203,9444922 mm³
Actual Dose expelled: 203,944 mm³ x 0,1 IU/mm³ = 20,39444922

In fig. 7C the bold dotted line shows variations in out-dosing compared to set values. As seen, the electronic motor drive compensation reduces the actual variations in out-dosing to far less than those of the reference example, even if mechanical tolerances have been doubled. As exemplified in the above calculation example variation cannot be corrected completely due to properties of the drive system *per se.*

### 2D: Example for drug delivery device with triple drug concentration and electronic piston rod drive

An exemplary drug delivery device has a cartridge, piston rod and drug concentration with dimensions and tolerances as set out in example 1E above, each increment of 1 IU corresponding to a piston rod rotation of 15 degrees. Corresponding to example 2C the drug delivery device is provided with an electronically controlled motor drive that will adjust the amount of piston rod rotation for a given nominally set dose size such that the expelled amount of drug corresponds to the set amount of drug.

Based on the above drug concentration, dimensions and tolerances deviations between dialled and expelled doses can be calculated and are illustrated in fig. 7D in which the full line represents standard and regulation requirements, the dotted line represents maximum device variations due to mechanical tolerances, and the bold dotted line shows variations in out-dosing compared to displayed values. As also seen, the electronic motor drive compensation reduces the actual variations in out-dosing to far less than those of the reference example, even if mechanical tolerances are maintained and the concentration tripled.

In the above description of exemplary embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

## Claims

1. A method of manufacturing a drug delivery device (200), comprising the steps of:
- providing a plurality of components (210, 220, 230) which in an assembled state form a processor-controlled drug delivery device, the drug delivery device comprising a drug-filled cartridge (230) or means for receiving a drug-filled cartridge, the cartridge comprising an outlet and an axially displaceable piston, the components comprising:
- a first group of components adapted to move during drug expelling, the first group of components comprising an axially displaceable piston drive member (220) adapted to engage the piston of a cartridge,
- a second group of components adapted to be stationary during drug expelling but interfacing with a member of the first group, and
- a processor with memory,
- determining a parameter value for at least one of: a member of the first group, and a member of the second group,
- assembling the components to form a drug delivery device, and
- storing in the processor memory correction data, the correction data being based on one or more differences between a determined parameter value and a corresponding nominal value.

2. A method of manufacturing a drug delivery device as in claim 1, wherein at least two parameter values are determined for a given member as a function of a given dimension or state, the correction data comprising a number of correction values varying as a function of a state of the drug delivery device.

3. A method of manufacturing a drug delivery device as in claim 1, wherein the plurality of components in the assembled state form a drug delivery device as defined in any of claims 8-11.

4. A method of manufacturing a drug delivery assembly (200), comprising the steps of:
- providing a drug-filled cartridge (230), the cartridge comprising an outlet and an axially displaceable piston (234),
- determining at least one parameter value for the cartridge,
- providing the cartridge with readable information in respect of the determined parameter value(s),
- providing a processor-controlled drug delivery device comprising:
- a cartridge holder adapted to receive the cartridge,
- means for reading the cartridge readable information,
- an expelling assembly, and
- a processor with memory

5. A method of manufacturing a drug delivery assembly as in claim 4, comprising the further steps of:
- inserting the cartridge in the cartridge holder,
- reading the cartridge readable information, and
- storing in the processor memory correction data, the correction data being based on difference(s) between the determined parameter value(s) and a corresponding nominal value(s).

6. A method of manufacturing a drug cartridge, comprising the steps of:
- providing a drug-filled cartridge (230), the cartridge comprising an outlet and an axially displaceable piston (234),
- determining at least one parameter value for the cartridge,
- providing the cartridge with readable information in respect of the determined parameter value(s).

7. A drug delivery device (200) comprising:
- a drug-filled cartridge (230) or means for receiving a drug-filled cartridge, the cartridge comprising an outlet and an axially displaceable piston,
- an expelling assembly comprising:
- a first group of components adapted to move during drug expelling, the first group of components comprising an axially displaceable piston drive member adapted to engage the piston of a cartridge,
- a second group of components adapted to be stationary during drug expelling but interfacing with a member of the first group,
- dose setting means (280) allowing a user to set a nominal dose amount of drug to be expelled,
- a display (202) adapted to display a dose amount,
- drive means (210) for moving the piston drive member in a distal direction to thereby expel drug from an inserted cartridge, and
- a processor comprising a memory, the processor being adapted to control the display to display a dose amount,
wherein:
- a correction value is stored in the memory, the correction value being based on one or more differences between a determined parameter value and a corresponding nominal value for at least one of: a member of the first group, a member of the second group, and a received cartridge, and
- the processor is adapted to, based on the correction value and a nominally set dose amount, calculate a corrected dose amount and control the display to display the corrected dose amount.

8. A drug delivery device as in claim 7, wherein at least two correction values are stored in the memory, the correction values being correlated to the position of the piston rod, and
- the processor is adapted to, based on the correction value for the current position of the piston rod and a nominally set dose amount, calculate a corrected dose amount and control the display to display the corrected dose amount.

9. A drug delivery device as in claim 8, wherein the display (202) is adapted to display dose amounts in increments of a given unit.

10. A drug delivery device as in claim 9, wherein the dose setting means is adapted to set a nominal dose amount of drug to be expelled in increments which are a fraction of the display increment.

11. A drug delivery device as in any of claims 8-10, comprising:
- a cartridge holder adapted to receive a cartridge, the cartridge being provided with readable information in respect of a determined parameter value for the cartridge, and
- means for reading the cartridge readable information,
wherein the processor is adapted to calculate a correction value based at least in part on the difference between the cartridge parameter value information and a corresponding stored nominal value.

12. A drug delivery device comprising:
- a drug-filled cartridge or means for receiving a drug-filled cartridge, the cartridge comprising an outlet and an axially displaceable piston,
- an expelling assembly comprising:
- a first group of components adapted to move during drug expelling, the first group of components comprising an axially displaceable piston drive member adapted to engage the piston of a cartridge,
- a second group of components adapted to be stationary during drug expelling but interfacing with a member of the first group,
- dose setting means allowing a user to set a nominal dose amount of drug to be expelled, the nominal dose amount corresponding to a nominal axial displacement for the piston drive member,
- a display adapted to display a dose amount,
- a motor for moving the piston drive member in a distal direction to thereby expel drug from an inserted cartridge, and
- a processor comprising a memory, the processor being adapted to control the motor to displace the piston drive member axially,
wherein:
- a correction value is stored in the memory, the correction value being based on one or more differences between a determined parameter value and a corresponding nominal value for at least one of: a member of the first group, a member of the second group, and
a received cartridge, and
- the processor is adapted to, based on the correction value and a nominally set dose amount, calculate a corrected axial displacement for the piston drive member and control the motor to move the drive member corresponding to the corrected axial displacement.

13. A drug delivery device as in claim 12, wherein at least two correction values are stored in the memory, the correction values being correlated to the position of the piston rod, and
- the processor is adapted to, based on the correction value for the current position of the piston rod and a nominally set dose amount, calculate a corrected axial displacement for the piston drive member and control the motor to move the drive member corresponding to the corrected axial displacement.
